# EUROPEAN PATENT APPLICATION

(11) **EP 3 158 868 A1**
(43) Date of publication of application: **26.04.2017**
(21) Application number: 16188581.9
(22) Date of filing: 03.12.2009
(51) Int. Cl.: A01N 65/22, A01N 65/24, A01N 59/16, A01P 1/00, A01N 65/00

(54) **INTRAMAMMARY TEAT SEALANT**

(30) Priority: 04.12.2008 US 119763 P
(62) Divisional of application: 09768574.7
(71) Applicant: Merial, Inc, Duluth GA 30096 (US)
(72) Inventor: RAZZAK, Majid, Auckland (NZ); HOLMES, Robert, Auckland (NZ); JOHNSON, Alan, Papatoetoe (NZ); GOSWAMI, Jitendra, Howick (NZ); AWASTHI, Atul, Manukau City (NZ)
(74) Representative: D Young & Co LLP

(57) **Abstract**

The present invention relates to veterinary or pharmaceutical formulations comprising one or more plant oils and bismuth subnitrate and methods for their manufacture and uses. The formulations are useful as a teat sealant to prevent or treat mastitis is an animal.

## Description

### CROSS-REFERENCE TO RELATED APPLICATIONS

This application claims benefit of US provisional application Serial No. 61/119,763 filed December 4, 2008.

### FIELD OF THE INVENTION

This invention relates to teat sealant formulations and their use for treating and preventing mastitis in an animal.

### BACKGROUND OF THE INVENTION

Mastitis is the most common disease of dairy cattle. In commercial herds, the cost due to reduced milk quality can be extremely significant. This cost can be due to reduced production and the need to withhold contaminated milk from the processing stream.

Mastitis is an inflammatory response of the udder tissue due to some form of injury, the most common being bacterial infection. The inflammatory response consists of an increase in blood proteins and white blood cells in the mammary tissue and the milk. The purpose of this response is to destroy the irritant, repair the damaged-tissue and return the udder to normal function.

Inflammation is characterized by: (a) swelling of the udder where persisting inflammation leads to tissue damage and replacement of secretory tissues within the udder with nonproductive connective tissues, (b) clotting of the milk, wherein these clots are congealed leukocytes, secretory cells and protein and (c) a lower milk yield.

Mastitis begins after bacteria pass through the teat canal and enter the part of the teat known as the cistern. There are two major periods during which this can occur: during the lactation period or during the non-laction (dry) period.

During the lactation period invasion of the teat usually occurs during milking. Organisms present in the milk or at the teat end enter the teat canal and cistern when there is admission of undesired air in the milking unit. After milking, the teat canal remains dilated for 1-2 hours while the canal of a damaged teat may remain partially open permanently. This makes it easier for organisms from the environment or those found on injured skin to enter the teat canal. Adherence of bacteria to tissues lining cisterns and ducts may prevent flushing-out during milking and help establish infections. Bacteria eventually enter the glandular tissues where they affect alveolar cells. Toxins produced by bacteria cause death of or damage to milk-secreting epithelial cells, and these cells produce substances to the blood stream that increase blood vessel permeability. This allows leukocytes to move from the blood into the alveolus where they function by engulfing bacteria.

At the conclusion of the lactation period and once milking has stopped for the season the teat canal is closed by the formation of a natural keratin teat plug. This typically happens over a period of 2-3 weeks. However prior to the formation of this teat plug the teat canal is open and highly susceptible to bacterial infection. It can also be the case that if the teat plug is poorly developed there is an opportunity for on-going infection. Indeed it takes between one and nine weeks for most cows to form this plug and up to 5% of cows never form one. Typically 50% of teats may still be "open" at 10 days after drying off (see, e.g., Williamson JH, Woolford MW, Day AM. The prophylactic effect of a dry cow antibiotic against Streptococcus uberis. New Zealand Veterinary Journal (1995) 43, 228-234).

To prevent new cases of mastitis during the dry period many farmers treat cows with a prophylactic treatment of an intramammary antibiotic. This is administered in the form of a paste or gel infused with antibiotic. A syringe is used to insert the material directly into the teat canal via the opening at the base of the teat. Prevention of mastitis is reliant on sufficient antibiotic being retained in the teat canal to kill off any bacteria that may enter the teat canal over the dry period.

However more recently there has been increased concern regarding the use of such antibiotic preventatives in dairy cows. This is due to two reasons: (a) the potential for antibiotic residues in milk, which can cause milk processors problems in producing culture-based dairy products and (b) the potential for the development of bacterial resistance to antibiotics crossing from animal to human strains.

An interesting solution to this issue has been the development of intramammaries designed to create a mechanical barrier in the teat canal that mimics the action of the cows natural keratin teat plug. By application of such a barrier the cow is potentially protected from the ingress of bacteria prior to the point at which the teat canal becomes sealed by the formation of the keratin plug.

Various means have been proposed by which this artificial mechanical barrier can be formed. These include: (a) film barriers (e.g., U.S. Patent No. 6,030,633) in which a film forming spray is applied to the outside and base of the teat, (b) physical plugs made of silicone and the like (e.g., U.S. Patent Publication No. 20070239181), which are designed to act as substrates for the formation of the teat plug, or to act as a teat plug in themselves and are inserted into the teat canal, (c) heavy metal inert pastes (e.g., U.S. Patent Nos. 6,506,400, 6,340,469 and 6,254,881), which are applied by syringe and once in the teat canal are designed to block the canal until expelled by the action of the new born calf sucking the teat, or by the farmer manually milking or "stripping-out" the paste.

In these cases, these barriers contain no antibiotic or anti-infective of any kind. The prevention of new infection is reliant simply on the integrity of the barrier formed across the teat opening or within the teat canal. However, just as the build up of milk pressure immediately following the cessation of milking at drying off may cause milk to leak from the teats and disrupt the formation of the natural keratin plug, this same event can lead to damage of these non-natural physical barriers.

In fact, x-ray imaging of the teats of heavy metal teat sealant treated cows 100 days after treatment revealed that at least a portion of seal material has "disappeared" (see, e.g., Woolford, M.W., Williamson, J.H., Day, A.M. and Copeman, P.J.A. (1998) The prophylactic effect of a teat sealer on bovine mastitis during the dry period and the following lactation. NZ Vet. J. 46: 12-19). The teat sealant material may have either fragmented and migrated to higher locations within the mammary gland. Or alternatively it may have been expressed via the teat canal due to build up of mammary pressure generated by movement of the cow or due to continued but reducing milk production.

There is, therefore, a chance that infection may get through the artificial physical barrier just as it does with the natural keratin plug.

Therefore, the need exists for a better teat plug product which does not facilitate the problems discussed above.

### SUMMARY OF THE INVENTION

Formulations comprising a plant oil and bismuth subnitrate, wherein the formulation is a gel or paste are provided. The formulations preferably contain natural anti-infective agents from plant oils and thus have anti-infective properties The formulations are useful for treating or preventing mastitis in udder tissue of an animal.

Methods of treating or preventing mastitis or inflammation of an udder tissue in an animal are also disclosed. The formulations disclosed herein can be contacted with udder tissue to prevent or treat mastitis.

Methods of preparing the formulations described herein are also provided. The formulations described herein can be prepared by any known method of contacting or mixing the components of the formulation to form a gel or paste.

### BRIEF DESCRIPTION OF DRAWINGS

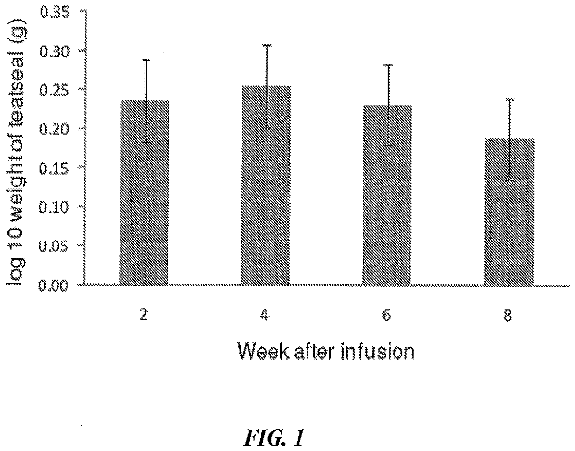

Figure 1 shows the mean (SEM) log₁₀ weight (g/gland) of teat sealant by week of infusion.

### DETAILED DESCRIPTION

The present invention relates to veterinary or pharmaceutical formulations comprising one or more plant oils and bismuth subnitrate and methods for their manufacture and uses. The formulations are useful as a teat sealant to prevent or treat mastitis in an animal,

The formulations described herein are beneficial by way of their ability to form a physical barrier to prevent ingress of new bacterial or fungal infection and actively prevent ingress of bacteria around or through the teat plug medium should the teat plug be less than perfectly formed. Ideally these additional defensive properties would not rely on an antibiotic, other than natural anti-infective agents, that could result in potential antibiotic residues in the cow or milk.

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicate otherwise.

The term "animal" is used herein to include all mammals, birds and fish and also include all vertebrate animals, including humans. It also includes an individual animal in all stages of development, including embryonic and fetal stages. As used herein, the term "livestock animals" refers to any breed or population of animal kept by humans for a useful or commercial purpose. For example, such animals include, but are not limited to, cattle (bovine), sheep (ovine), pigs (porcine or swine), poultry (avian), and the like. As used herein, the term "cow" or "cattle" is used generally to refer to an animal of bovine origin of any age. Interchangeable terms include "bovine", "calf", "steer", "bull", "heifer", "cow" and the like. As used herein, the term "pig" is used generally to refer to an animal of porcine origin of any age. Interchangeable terms include "piglet", "sow" and the like.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

This invention is based, in part, on Applicants' discovery of formulations of low levels of a natural plant oil extract known to have anti-infective properties with heavy metal sealant base material in such a manner that the oil does not have a tendency to migrate from the sealant and into the surrounding tissues.

In an embodiment, the present invention relates to a teat sealant formulation which comprises at least one plant oil and bismuth subnitrate, wherein the formulation is a gel or a paste. The gel or paste may further comprise liquid paraffin, aluminum stearate and/or silicon dioxide. The plant oil is selected from the group consisting of thyme (wild) oil, thyme (red) oil, thyme (geraniol) oil, cinnamon bark oil, cinnamon leaf oil, and a mixture thereof. The plant oil may be present in an amount of about 1% to about 15% w/w, about 1% to about 14% w/w, about 1% to about 13% w/w, about 1% to about 10% w/w, about 2% to about 12% w/w, about 2% to about 11% w/w, about 2% to about 10% w/w, about 3% to about 9% w/w, about 3% to about 8% w/w, about 4% to about 8% w/w, about 4% to about 7% w/w, about 4% to about 6% w/w, about 1% to 5% w/w, about 1% w/w, about 1.5% w/w, about 2% w/w, about 2.5% w/w, about 3% w/w, about 3.5% w/w, about 4% w/w, about 4.5% w/w, or about 5% w/w. Further, the plant oil may be cinnamon bark or cinnamon leaf oil, which may be present in an amount of about 1% to about 10% w/w, or about 1% to 5% w/w, or about 2.5% to 5% w/w. In another embodiment, the plant oil may be thyme oil, which may be present in an amount of about 1% to about 10% w/w, about 1% to 5% w/w, or about 1% to 3% w/w.

One embodiment of the invention is the addition of low levels of a natural plant oil extract known to have anti-infective properties to a heavy metal sealant base in such a manner that the oil does not have a tendency to migrate from the sealant and into the surrounding tissues.

There are potentially many oils that could serve this purpose. The oil is extracted from the seed, bark, flower, root or other part of the plant. A non-limiting list of some of the plant extract oils that have been shown to have anti-infective properties that could be used in the present invention includes: Akebia (Akebia trifoliate), Alfalfa (Medicago sativa),Alkanet (Alkanna tinctoria), Allegheny Barberry (Berberis Canadensis), Almond (Prunis amygdalis), Ampelopsis (Ampelopsis japonica), Amur Barberry (Berberis amurensis), Amur Cork Tree (Phellodendron amurense), Amyris (Amyris blsamifera), Angelica (Angelica anomala), Aniseed (Pimpinella anisum), Annual Yellow Sweetclover (Melilotus indicus), Apple (Malus domestica), Arnebia (Arnebia euchroma), Australian Sandalwood (Santalum sicatum),Bai Zhi (Angelica dahurica), Bai Zhu (Atractylodes macrocephala), Baikal Skullcap (Scutellaria baicalensis), Barberry (Berberis georgii), Barberry (Berberis vulgaris),Barrenwort (Epimedium grandiflorum), Basil (Ocimum basilicum), Bay (Pimenta racemosa), Bay Laurel (Lauri fructus), Bay Laurel Leaf Oil (Lauri folii aetheroleum), Bei Chai Hu (Bupleurum chinense), Bei Sha Shen (Glehnia littoralis), Benzoin (Lindera benzoin), Benzoin (Styrax benzoin), Bergamot (Citrus bergamia), Biota (Thuja orientalis), Berberis spp., Birch (Betula alba), Birch (Betula lenta), Bitter Almond (Prunus dulcis), Bitter Fennel (Foeniculum vulgare), Bitter Orange (Citrus aurantium), Black Medick (Medicago lupulina), Black pepper (Piper nigrum), Black-berried Barberry (Berberis calliantha), Blue Fruited Barberry(Berberis gagnepainii), Bog Myrtle (Myrica gale), Boldo leaf (Boldo folium), Borage starflower (Borago officinalis), Box Thorn (Lycium barbarum), Bu Gu Zhi (Psoralea corylifolia), Buchu (Barosma betulina), Buckthorn Bark (Frangulae cortex), Burning Bush (Dictamnus albus), Cade (Juniperus oxycedrus), Cajuput (Melaleuca leucadendra), Cajuput (Melaleuca leucadendron), Calamus Root (Acorus calamus), California Barberry (Mahonia pinnata), Camomile (Matricaria recutita), Camomile Flower (Matricariae flos), Camphor (Cinnamomum camphora), Caraway fruit (Carum carvi), Caraway oil (Carvi aetheroleum), Cardamom (Elettaria cardamomum), Carnation (Dianthus caryophyllus), Carrot Seed (Daucus carota), Castor (Ricinus communis), Cedarwood (Juniperus virginiana), Cedarwood Atlas (Cedrus atlantica), Celery (Apium graveolens), Chamomile Maroc (Ormenis multicaulis), Champaka (Michelia champaka), Che Qian Zi (Plantago asiatica), Cherokee Rose (Rosa laevigata), Chinese Asparagus (Asparagus cochinchinensis), Chinese Barberry (Berberis chinensis), Chinese Boxthorn (Lycium chinense), Chinese Cork Tree (Phellodendron chinense), Chinese Haw (Crataegus pinnatifida), Chinese Mahonia (Mahonia fortunei), Chinese Motherwort (Leonurus sibiricus), Chinese Peony (Paeonia lactiflora), Chinese Pink (Dianthus chinensis), Chinese Water Chestnut (Eleocharis dulcis), Chitra Barberry (Berberis aristata), Chrysanthemum (Dendranthema x grandiflorum), Cinnamon (Cinnamomum zeylanicum), Cinnamon bark (Cinnamonum cassia), Citronella (Cymbopogon nardus), Citronella Oil (Citronellae aetheroleum), Clary Sage (Salvia sclarea), Clematis mandschurica, Clove (Eugenia caryophyllata), Clove (Syzygium aromaticum), Clove Oil (Caryophylli Aetheroleum), Cocklebur (Xanthium strumarium), Coconut (Cocos nucivera), Cogongrass (Imperata cylindrica), Colorado Barberry (Berberis fendleri), Common Cockscomb (Celosia argcntea cristata), Condurango Bark (Condurango cortex), Coptis japonica, Coriander (Coriandrum sativum). Coriander Bark (Coriandri aetheroleum), Cornus spp., Costus (Saussurea costus), Costus Root (Sassuriea costus), Cottonseed (Gossypium herbaceum), Crab Apple (Malus sylvestris), Creeping Oregon Grape (Mahonia repens), Cnidium officinale, Cumin (Cuminum cyminum), Cypress (Cupressus sempervirens), Cypress Oil (Cupressi aetheroleum), Cyrtomium fortunei, Da Ji (Euphorbia pekinensis), Damiana (Turnera diffusa), Darwin's Barberry (Berberis darwinii, Day Flower (Commelina communis), Dill (Anethum graveolens), Dutch Rush (Equisetum hyemale), Dwarf Barberry (Mahonia pumila), Elder Flower (Sambuci flos), Elecampane (Inula helenium), Elemi (Canarium luzonicum), Elsholtzia ciliata, Eucalyptus leaf (Eucalyptus globules), Eucalyptus Oil (Eucalypti aetheroleum), Evening Primrose (Oenothera biennis), Fennel (Foeniculi aetheroleum), Fennel (Gentianae cortex), Fir (Abies balsamea), Flax-Leaved Paper-Bark (Melaleuca linariifolia), Fodder Radish (Raphanus sativus oleiformis), Foetid Bugbane (Cimicifuga foetida), Frangipani (Plumeria rubra), Frankincense (Boswellia carteri), French Rose (Rosa gallica), Fringed Pink (Dianthus superbus), Frogfruit (Phyla nodiflora), Fumewort (Corydalis solida), Galangal (Alpinia officinarum), Galbanum (Ferula galbaniflua), Gardenia (Gardenia grandiflora), Garlic Chives (Allium tuberosum), Gayfeather (Liatris spicata), Gentiana spp., Geranium (Pelargonium graveolens), Ginger (Zingiber officinale), Gleditsia sinensis, Golden Bells (Forsythia viridissima), Goldenseal (Hydrastis canadensis), Goldthread (Coptis trifolia), Grapefruit (Citrus paradisii), Grass-leaved Sweet Rush (Acorus gramineus), Great Burdock (Arctium lappa), Great Water Plantain (Alisma plantago-aquatica), Hairy Agrimony (Agrimonia pilosa), He Shou Wu (Polygonum multiflorum), Helichrysum, Immortelle (Helichrysum angustifolium), Hemp (Cannabis sativa), Henna (Lawsonia inermis), Himalayan Barberry (Berberis angulosa), Holy Basil (Ocimum sanctum), Honeysuckle (Lonicera caprifolium), Hop (Humulus lupulus), Hop Tree (Ptelea trifoliata mollis), Horseradish (Armoracia rusticana), Hou Po (Magnolia officinalis), Hu Huang Lian (Picrorhiza scrophulariiflora), Huang Jing (Polygonatum sibiricum), Huang Lian (Coptis chinensis), Huang Ping (Vitex negundo), Huang Qi (Astragalus membranaceus), Hyacinth (Hyacinthus orientalis), Hyacinth Orchid (Bletilla striata), Hyssop (Hyssopus officinalis), Indian Pipe (Monotropa uniflora), Indian Poke (Phytolacca acinosa), Ilex pubescens, Inula (Inula graveolens), Ivy (Hedera helix), Japanese Apricot (Prunus mume), Japanese Barberry (Berberis thunbergii), Japanese Honeysuckle (Lonicera japonica), Japanese Pagoda Tree (Sophora japonica), Japanese Pepper Tree (Zanthoxylum piperitum), Japanese Privet (Ligustrum japonicum), Jasmine (Jasminium grandiflorum), Jasmine (Jasminum officinale), Juniper (Juniperus communis), Juniper Berry (Juniperi fructus), Komarov's Bugbane (Cimicifuga heracleifolia), Korean Barberry (Berberis koreana), Korean Mint (Agastache rugosa), Korean Nut Pine (Pinus koraiensis), Korean Pasque Flower (Pulsatilla koreana), Ku Shen (Sophora flavescens), Kuru (Picrorhiza kurroa), Labdanum (Cistus ladaniferus), Laurel (Laurus nobilis), Lavander (Lavandula angustifolia), Lavender (Lavandulae aetheroleum), Leatherleaf Mahonia (Mahonia bealei), Lemon (Citrus limonum), Lemon Oil (Citri aetheroleum), Lemon Tea Tree (Leptospermum petersonii), Lemon Verbena (Lippia citriodora), Lemongrass (Cymbopogon flexuosus), Lemon-Scented Gum (Eucalyptus citriodora), Lemon-Scented Tea Tree (Leptospermum liversidgei), Leonurus japonicus, Leopard Lily Belamcanda chinensis), Lepidium apetalum, Lesser Burdock (Arctium minus), Lime (Citrus aurantifolia), Linaloe (Bursera delpechiana), Linden Blossom (Tilia cordata), Linden Flower (Tiliae flos), Linseed (Linum usitatissimum), Linseed Oil (Lini oleum), Litsea (Litsea cubeba), Loquat (Eriobotrya japonica), Lotus (Nelumbo nucifera), Ma Dou Ling (Aristolochia debilis), Manuka (Melaleuca alternifolia), Magellan Barberry (Berberis buxifolia), Mahoberberis aquisargentii, Mahonia spp., Maidenhair Tree (Ginkgo biloba), Maize (Zea mays), Majoram (Majorane herba), Male Fern (Dryopteris filix-mas), Mandarin (Citrus rticulata), Marjoram (Thymus mastichina), Melissa (Melissa officinalis), Melissa Leaf (Melissae folium), Melissa Oil (Melissae aetheroleum), Mexican Barberry (Mahonia haematocarpa), Mexican Barberry (Mahonia trifoliolata), Mimosa (Acacia dealbata), Mint Bush (Prostanthera rotundifolia), Mint Oil (Menthae arvensis aetheroleum), Moutan (Paconia suffruticosa), Mustard (Brassica juncea), Myrrh (Balsamodendron myrrha), Myrrh (Commiphora myrrha), Myrtle (Myrtus communis), Nasturtium (Tropaeolum majus), Nepalese Crane's Bill (Geranium nepalense), Neroli (Citrus aurantium), Niaouli [Melaleuca viridiflora), Nut Grass (Cyperus rotundus), Nutmeg (Myristica fragrans), Oak Bark (Quercus cortex), Oak Moss (Evernia prunastri), Olive (Olea europaea), Orange Blossom (Citrus cinensis blossom), Oregano (Origanum valgarc), Oregon Grape (Mahonia aquifolium), Oregon Grape (Mahonia nervosa), Oriental Radish (Raphanus sativus niger), Oriental Sweet Gum (Liquidambar orientatis), Origanum (Thymus capitatus), Qsmanthus (Osmanthus fragrans), Paeonia spp., Palmarosa (Cymbopogon martinii), Panax pseudoginseng, Paprika (Capsici fructus acer), Paradise Apple (Malus pumil), Parsley (Petroselinum sativum), Patchouly (Pogostemon patchouli), Patrinia spp., Peanut (Arachis hypogaea), Pei Lan (Eupatorium japonicum), Pennycress (Thlaspi arvense), Pennyroyal (Mentha pulegium), Peppermint (Mentha piperita), Peppermint Bark (Menthae piperitae aetheroleum), Peru Balsam (Miroxylon balsamum), Petitgrain (Citrus aurantium), Pine Needle (Pinus sylvestris), Pink Fruited Barberry (Berberis soulieana), Pipsissewa (Chimaphila umbellate), Pomegranate (Punica granatum), Potato (Solanum tuberosum), Purple Shiso (Perilla frutescens nankinensis), Phytolacca esculenta, Qian Hu (Peucedanum praeruptorum), Qing Hao (Artemisia annua), Radish (Raphanus sativus), Rapeseed (Brassica napus), Rat-Tail Radish (Raphanus sativus caudatus), Ravensara (Ravensara aromatica), Red Thyme (Thymus serpyllum), Rhododendron (Rhododendron anthopogon), Rhubarb Root (Rhei radix), Ribwort Plantain (Plantago lanceolata), Rocket (Eruca vesicaria sativa), Roman Chamomile (Anthemis nobilis), Rose (Rosa damascena), Rose Geranium (Pelargonium rosa), Rose Musk (Rosa mochata), Rosehip (Rosa rubiginosa), Rosemary (Rosmarinus officinalis), Rosemary Leaf (Rosmarini folium), Rosemary Oil (Rosmarini aetheroleum), Rosewood (Aniba rosaedora), Roundheaded Bush Clover (Lespedeza capitata), Rue (Ruta graveolens), Safflower (Carthamnus tinctorius), Safflower (Carthamus tinctorius), Saffron (Crocus Sativus), Sage (Salvia officinalis), Sage (Salviae aetheroleum), Sage Leaf (Salviae folium), Salmon Barberry (Berberis aggregate), Sandalwood (Santalum album), Sargentodoxa cuneata, Schisandra (Schisandra chinensis), Scrophularia ningpoensis, Self-Heal (Prunella vulgaris), Sesame (Sesamum Indicum), Shan Zhu Yu (Cornus officinalis), Shiso (Perilla frutescens), Shoo Fly (Nicandra physaloides), Siberian Yarrow (Achillea sibirica), Snake-Needle Grass (Oldenlandia diffusa), Soap Tree (Quillaia saponins), Spanish Needles (Bidens bipinnata), Spanish sage (Salvia lavandulaefolia), Spearmint (Mentha spicata), Spike Lavender (Lavandula latifolia), Spikenard (Nardostachys jatamansi), Spotted Wintergreen (Chimaphila maculate), Spruce (Tsuga Canadensis), Star Anise (Illicium anisatum), Star anise (Illicium Verum), Star Jasmine (Trachelospermum jasminoides), Stinging Nettle Herb (Urticae herba), Strawberry Saxifrage (Saxifraga stolonifera), Summer Cypress (Bassia scoparia), Sundew (Drosera rotundifolia), Sunflower (Helianthus divaricatus), Sweet Majoram (Origana majorana), Sweet orange (Citrus senensis), Tagetes (Tagetes glandulifera), Tansy (Tanacetum vulgare), Taraxacum spp., Tarragon (Artemisia dracunculus), Tarragon (Artemisia scoparia), Tartarian Aster (Aster tataricus), Tea Tree (Melaleuca alternifolia), Texas Mahonia (Mahonia swaseyi), Thorow-Wax (Bupleurum falcatum), Thyme (Thymus vulgaris), Thyme Oil (Thymi aetheroleum), Thyme-Leaved Savory (Satureja thymbra), Tian Nan Xing (Arisaema consanguineum), Tree Of Heaven (Ailanthus altissima), Tree Peony (Paeonia spp.), Trichosanthes kirilowii japonica, Tsi (Houttuynia cordata), Tuberose (Polianthes tuberosa), Tumeric (Curcuma Longa), Tung Tree (Aleurites fordii), Valerian (Valeriana officinalis), Vanilla (Vanilla planifolia), Velvet Barberry (Berberis tomentosa), Vervain (Verbena officinalis), Vetiver (Vetiveria zizanoides), Violet Leaf (Viola odorata), Wax Myrtle (Myrica cerifera), Welsh Onion (Allium fistulosum), White Mulberry (Morus alba), White Mustard (Sinapis alba), Wild Ginger (Asarum sieboldii), Wild Indigo (Baptisia tinctoria), Wintergreen (Gaultheria procumbens), Woad (Isatis tinctoria), Wu Jia Pi (Eleutherococcus gracylistylus), Xing An Sheng Ma (Cimicifuga dahurica), Xuan Fu Hua (Inula britannica), Yama-Shakuyaku (Paeonia japonica), Yarrow (Achillea ,illefolium), Yarrow (Millefolii herba), Yin Chen Hao (Artemisia capillaries), Ylang Ylang (Cananga odorata), Yun Lian (Coptis teeta), Yuzu (Citrus junos), Zinziba (Lippia javanica).

Each oil contains a mix of constituents that may have a range of clinical effects such as anti-bacterial or anti-fungal.

**Table 1. Approximate Constituents of Essential Oils**

| **Essential oil** | **Mayor constituent (content)** |
|---|---|
| Cinnamon bark oil | cinnamaldehyde (63.1%) |
| Lemongrass oil | neral (33.2%), geranial (37.8%) |
| Perilla oil | limonene (18.9%), perillaldehyde (60.8%) |
| Thyme (wild) oil | carvacrol (80.0%) |
| Thyme (red) oil | limonene (25.8%), γ-terpinene (19.4%), thymol (25.5%) |
| Thyme (geraniol) oil | geraniol (32.7%), geranyl acetate (55.6%) |
| Peppermint oil | *p*-menthone (19.5%), menthol (63.5%) |
| Tea tree oil | γ-terpinene (17.7%), terpinen-4-ol (42.5%) |
| Coriander oil | linalool (73.2%) |
| Lavender (spike) oil | 1,8-cineole (23.7%), linalool (46.3%), camphor (17.1%) |
| Lavender (true) oil | linalool (30.1%), linalyl acetate (36.6%) |
| Rosemary oil | α-pinene (24.1%), 1,8-cineole (23.5%), camphor (19.7%) |
| Eucalptus (radiata) oil | 1,8-cineole (74.3%), α-terpineol (10.3%) |
| Citron oil | limonene (83.1 %) |

The formulation may comprise bismuth, such as bismuth subnitrate, or heavy bismuth subnitrate. The present invention further comprises other bismuth salts, such as but not limited to, bismuth oxychloride, bismuth subcarbonate, bismuth subgallate, bismuth subsalicylate or bismuth telluride. In one embodiment, bismuth, such as bismuth subnitrate, may be present in an amount of about 40% to about 90%w/w, about 45% to about 85% w/w, about 50% to about 80% w/w, about 55% to about 75% w/w or about 60% to about 70% w/w. In another embodiment, the bismuth subnitrate may be present in an amount of about 50% to about 70% w/w.

The present invention may also comprise liquid paraffin. Liquid paraffin, or mineral oil, is a mixture of heavier alkanes, and has a number of names, including nujol, adepsine oil, alboline, glycol, medicinal paraffin, saxol, or USP mineral oil. In one embodiment, liquid paraffin may be present in an amount of about 5% to about 50% w/w, about 10% to about 45% w/w, about 15% to about 40% w/w, about 20% to about 35% w/w or about 25% to about 30% w/w. In another embodiment, the liquid paraffin may be present in an amount of about 25% to about 40% w/w.

The present invention may also comprise a stearate salt, such as but not limited to, aluminum stearate, ammonium stearate, barium stearate, butyl stearate, cadmium stearate, calcium stearate, cobalt stearate, copper stearate, glycol stearate, lithium stearate, magnesium stearate, manganese stearate, methyl stearate, sodium stearate, strontium stearate or zinc stearate. In one embodiment, the stearate salt is aluminum stearate. The stearate salt, such as aluminum stearate, may be present in an amount of about 0.1 % to about 10%, about 1% to about 5%, about 2% to about 4% w/w, about 2% to about 3% w/w, about 2.1% to about 2.9% w/w, about 2.1% to about 2.8% w/w, about 2.2% to about 2.7% w/w, about 2.2% to about 2.6% w/w, about 2.3% to about 2.5% w/w, about 2.3% to about 2.4% or about 2.3% w/w. In yet another embodiment, the aluminum stearate may be present in an amount of about 1.5% to about 4% w/w.

In an alternate embodiment wherein the milk from a cow is ultimately used in cheese production, the formulation may comprise a bismuth-free, non-toxic heavy metal salt in a gel base (see, e.g., WO 2008/045920). This bismuth-free formulation especially useful in milk processing into cheese wherein a visual defect, termed "black spot defect" (BSD), takes the form of small, black spots (roughly 0.5 to 5 mm in diameter) that appear throughout the aged cheese. The spots are a purely aesthetic, visual defect that lowers the grade quality (and hence the market value) of the cheese affected with the problem. The spots are not accompanied by any organoleptic defect in the cheese. Cheese affected with the black spots is saleable, albeit at a lower grade than unaffected cheeses. In this embodiment, the formulation may comprise a plant oil carrier, liquid paraffin, aluminum stearate, silicon dioxide and a non-toxic heavy metal salt, such as but not limited to, titanium dioxide, zinc oxide, barium sulfate or any combination thereof.

Useful thickeners for the formulations described herein are well known in the art. Compounds which function as thickeners include, for example, celluloses, starches, natural gums, monothioglycerol, synthetic polymers, such as polymers and copolymers of polyvinylpyrrolidone or (meth)acrylates, etc. In one embodiment, thickeners include one or more of sodium carboxymethylcellulose (CMC), hydroxypropylcellulose, xanthum gum and hydroxyethyl starch. Thickeners may be present in amounts of from about 0.1 % to about 30 %. In another embodiment, the thickener is silica, such as a silicon dioxide. In yet another embodiment, the silicon dioxide is at a concentration of about 0.1% w/w, about 0.2% w/w, about 0.3% w/w, about 0.4% w/w, about 0.5% w/w, about 0.6% w/w, about 0.7% w/w, about 0.8% w/w, about 0.9% w/w, about 1% w/w, about 2% w/w, about 3% w/w, about 4% w/w or about 5% w/w. In yet another embodiment, the silicon dioxide may be present in an amount of about 0.1% to about 2% w/w.

The gel base can be any suitable gel formulation, a host of which are known in the pharmaceutical arts. The preferred gel base comprises aluminum stearate and liquid paraffin (e.g. mineral oil, white petrolatum, yellow petrolatum, etc.). Typical gel bases include a wax or oil of some type, and a salt such as aluminum or magnesium stearate. In another embodiment of the invention, the gel comprises a polyethylene gel. The gel may be based on low density polyethylene or on high density polyethylene.

Opacifiers may be added to modify rheology and may thus enhance the stability of the formulations. Opacifiers include, for example, zinc oxide or titanium dioxide and may be present in amounts from about 0.5% to about 2.5 %. Titanium dioxide is especially preferred. These compounds are well known to practitioners of this art.

Additionally, the formulations described herein may contain other inert ingredients such as antioxidants and preservatives. These compounds are well known in the formulation art. Antioxidant such as an alpha tocopheral, ascorbic acid, ascrobyl palmitate, fumaric acid, malic acid, sodium ascorbate, sodium metabisulfate, n-propyl gallate, BHA (butylated hydroxy anisole), BHT (butylated hydroxy toluene) monothioglycerol and the like, may be added to the present formulation. The antioxidants are generally added to the formulation in amounts of from about 0.01% to about 2.0 %, based upon total weight of the formulation, with about 0.05% to about 1.0 % being especially preferred. Preservatives, such as the parabens (methylparaben and/or propylparaben), are suitably used in the formulation in amounts ranging from about 0.01% to about 2.0 %, with about 0.05% to about 1.0 % being especially preferred. Other preservatives include benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, and the like. Ranges for these compounds include from about 0.01% to about 5 %.

Colorants may be added to the inventive formulations. Colorants contemplated by the present invention are those commonly known in the art. Specific colorants include, for example, dyes, an aluminum lake, caramel, colorant based upon iron oxide or a mixture of any of the foregoing. Especially preferred are organic dyes and titanium dioxide. Preferred ranges include from about 0.5 %% to about 25 %.

The formulation of the present invention may be packaged in a syringe filled with a single dose of the inventive teat sealant. The dosage may be about 1g, about 2g, about 3g, about 4g, about 5g, about 6g, about 7g, about 8g or about 9g. In one embodiment, the formulation of the present invention is applied by syringe and is designed to block the canal once in the teat canal until expelled by the action of the new born calf sucking the teat, or by the farmer manually milking or "stripping-out" the paste or gel.

The present invention also encompasses methods of treating an animal to prevent or treat mastitis, comprising administering to the animal an effective amount of any of the formulations described herein. Preferably, the udder tissue of the animal is contacted with a formulation as described herein. The formulation can be injected or infused into the udder tissue. The udder tissue includes all tissues and structures of the teat, including the teat canal and cistern and surrounding tissues. "Treatment" or "treating" is defined as the application or administration of a composition of the invention wherein the animal has mastitis, a symptom of mastitis, or a predisposition towards mastitis, where the purpose is to cure, heal, alleviate, relieve, alter, ameliorate, improve, or affect the disease, the symptoms of the disease, or the predisposition toward the disease.

The present invention also encompasses methods of preventing or reducing inflammation in udder tissue of an animal comprising contacting the tissue with a formulation as described herein. In an embodiment, the formulation controls the growth of bacteria, particularly *Staphylococcus aureus*, *Streptococcus uberis* and *E. Coli*. The terms "prevent" and "preventing" as used herein refer to the treatment of an animal that is at the time of treatment asymptomatic for mastitis or inflammation of udder tissues.

The present invention also encompasses methods of preparing the formaulations described herein. The components of the formulations are contacted or mixed to form a gel or paste. Any known method of formulating a gel or paste is useful in preparing the formulations described herein.

The invention is further described by the following non-limiting examples which further illustrate the invention, and are not intended, nor should they be interpreted to, limit the scope of the invention.

### EXAMPLES

### Example 1. Teat Sealant Base formulation

To test the ability of potential plant based oils to work in the required manner a base formulation was developed. This formulation was as follows:

**Table 2: Teat Sealant**

| **Material** | **% w/w** |
|---|---|
| Liquid Paraffin | 25-30% |
| Aluminum stearate | 2.3 |
| Silicon Dioxide | 1 |
| Plant Oil Carrier | 1-10% |
| Bismuth Subnitrate (Heavy) | To volume |

The formulation was prepared by the manufacturing method described below.

**Table 3: Manufacturing Method**

| | **Teat Sealant** |
|---|---|
| 1 | Load Liquid Paraffin and start heating |
| 2 | Add Aluminum stearate |
| 3 | Continue heating with mixing |
| 4 | Cool and add part of the Bismuth subnitrate |
| 5 | Continue mixing add remaining bismuth subnitrate |
| 6 | Add Silicon Dioxide, Plant Oil Extract and continue mixing |

Test samples of Teat Sealant containing Bismuth subnitrate and either peppermint oil or 2.5% and 5% cinnamon bark oil and 2.5% cinnamon leaf oil were prepared.

*In vitro* tests were conducted to determine: the natural anti-infective agent might be most suited to incorporation in a teat sealing medium; the level of agent that would be appropriate; the activity the agents may have when compared to a known antibiotic based intramammary formulation (CEFAMASTER®, Cefalonium Paste 8.3%w/w, Merial, Duluth, GA).

A commercially available teat sealant product (TEATSEAL®, Bismuth subnitrate 65%w/w, Pfizer, New York, NY) containing no anti-infective agent was also tested as a negative control. The result of this test showed that, as expected there was no anti-infective activity demonstrated by the product (Table 4).

The methodology used for testing was based on AATCC Test Method 147-1998 (Antibacterial Activity Assessment of Textile Materials: Parallel Streak Method).

Test organisms used in the various tests were:
*Staphylococcus aureus* ATCC 6538
*Streptococcus uberis* ATCC 19436
*Escherichia coli* ATCC 8739

The test organisms were subcultured from a stock culture onto appropriate maintenance medium as required. From these, a suspension was prepared in sterile 0.85% sodium chloride solution to a density visually equivalent to that of a McFarland standard of 1. These bacterial broth suspensions were used to streak parallel lines on the test media with a sterile cotton swab.

50ml of Tryptic Soy Agar nutrient medium (TSA) was dispensed into 135mm petri dishes. Each plate was inoculated with the appropriate organism with 3 parallel streaks.

Approximately 0.1 gram of the test product was applied evenly across the surface of the Aluminum foil carrier. Preliminary testing revealed the Aluminum foil had no antimicrobial effect against the test organisms. The carrier was then placed perpendicular to the parallel streaks of the test organisms on the media.

Antibacterial activity was measured by the presence or absence of clear zones around the carrier containing the product, and the presence or absence of growth beneath the carrier. All products and controls were performed in duplicate.

The zone measurement refers to the total width of the carrier with test specimen and any clear zone either side in mm. If no clear zone was observed, a result of 0 mm was recorded.

**Table 4: Antibacterial Activity of Commercial Teat Sealants**

| | | ***Staphylococcus aureus* ATCC6538** | | ***Streptococcus uberis* ATCC19436** | |
|---|---|---|---|---|---|
| **Sample Description** | | **Zone Size (mm)** | **Growth/No Growth Under Carrier** | **Zone Size (mm)** | **Growth/No Growth Under Carrier** |
| TEATSEAL® | **1** | 0 | Growth | 0 | Growth |
| | **2** | 0 | Growth | 0 | Growth |
| | **3** | 0 | Growth | 0 | Growth |
| TEATSEAL® | **1** | 0 | Growth | 0 | Growth |
| | **2** | 0 | Growth | 0 | Growth |
| | **3** | 0 | Growth | 0 | Growth |

### Example 2. Teat Sealant Formulations Containing Peppermint

Tests were conducted on teat sealant formulations containing peppermint oil, a material known for its anti-infective properties. These were compared with the commercially available CEFAMASTER® paste formulation. In this test, the samples did not demonstrate any antimicrobial activity against the *Staphylococcus aureus* and *Streptococcus uberis* strains used during the analysis.

**Table 5: Antibacterial Activity of Teat Sealants with Peppermint Oil**

| | | ***Staphylococcus aureus* ATCC6538** | | ***Streptococcus uberis* ATCC19436** | |
|---|---|---|---|---|---|
| **Sample Description** | | **Zone Size (mm)** | **Growth/No Growth under Aluminum Foil Carrier** | **Zone Size (mm)** | **Growth/No Growth under Aluminum Foil Carrier** |
| 1% Peppermint Oil | **1** | 0 | Growth | 0 | Growth |
| | **2** | 0 | Growth | 0 | Growth |
| | **3** | 0 | Growth | 0 | Growth |
| 1% Peppermint Oil | **1** | 0 | Growth | 0 | Growth |
| | **2** | 0 | Growth | 0 | Growth |
| | **3** | 0 | Growth | 0 | Growth |
| 2% Peppermint Oil | **1** | 0 | Growth | 0 | Growth |
| | **2** | 0 | Growth | 0 | Growth |
| | **3** | 0 | Growth | 0 | Growth |
| 2% Peppermint Oil | **1** | 0 | Growth | 0 | Growth |
| | **2** | 0 | Growth | 0 | Growth |
| | **3** | 0 | Growth | 0 | Growth |
| 3% Peppermint Oil | **1** | 0 | Growth | 0 | Growth |
| | **2** | 0 | Growth | 0 | Growth |
| | **3** | 0 | Growth | 0 | Growth |
| 3% Peppermint Oil | **1** | 0 | Growth | 0 | Growth |
| | **2** | 0 | Growth | 0 | Growth |
| | **3** | 0 | Growth | 0 | Growth |
| CEFAMASTER® (cephalonium 8.3%) | **1** | 48 | No Growth | 56 | No Growth |
| | **2** | 49 | No Growth | 56 | No Growth |
| | **3** | 49 | No Growth | 56 | No Growth |
| CEFAMASTER® (cefalonium 8.3%) | **1** | 49 | No Growth | 53 | No Growth |
| | **2** | 48 | No Growth | 54 | No Growth |
| | **3** | 49 | No Growth | 56 | No Growth |

Tests were then conducted to determine whether a higher level of peppermint oil may produce a better result. This 5% material was tested against a 100% peppermint oil sample.

**Table 6: Antibacterial Activity of Teat Sealants with Peppermint Oil**

| | | ***Staphylococcus aureus* ATCC6538** | | ***Streptococcus uberis* ATCC19436** | | ***Escherichia coli* ATCC8739** | |
|---|---|---|---|---|---|---|---|
| **Sample Description** | | **Zone Size (mm)** | **Growth/No Growth Under Carrier** | **Zone Size (mm)** | **Growth/No Growth Under Carrier** | **Zone Size (mm)** | **Growth/No Growth Under Carrier** |
| 5% Peppermint oil | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |
| 5% Peppermint oil | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |
| 100% peppermint oil | **1** | 42 | No Growth | 38 | No Growth | 0 | Growth |
| | **2** | 41 | No Growth | 38 | No Growth | 0 | Growth |
| | **3** | 41 | No Growth | 38 | No Growth | 0 | Growth |
| 100% peppermint oil | **1** | 41 | No Growth | 41 | No Growth | 0 | Growth |
| | **2** | 43 | No Growth | 43 | No Growth | 0 | Growth |
| | **3** | 39 | No Growth | 49 | No Growth | 0 | Growth |

The peppermint oil did not show a satisfactory result at the increased level.

### |Example 3. Teat Sealant Formulations Containing Cinnamon Bark Oil or Cinnamon Leaf Oil

Samples of a teat sealant medium were prepared incorporating varying levels of cinnamon leaf or cinnamon bark oil.

**Table 7: Antibacterial Activity of Teat Sealants with Cinnamon Oil**

| | | ***Staphylococcus aureus* ATCC6538** | | ***Streptococcus uberis* ATCC19436** | | ***Escherichia coli* ATCC8739** | |
|---|---|---|---|---|---|---|---|
| **Sample Description** | | **Zone Size (mm)** | **Growth/No Growth Under Aluminum Foil Carrier** | **Zone Size (mm)** | **Growth/No Growth Under Aluminum Foil Carrier** | **Zone Size (mm)** | **Growth/No Growth Under Aluminum Foil Carrier** |
| 2.5% cinnamon leaf oil | **1** | 0 | No Growth | 0 | No Growth | 0 | No Growth |
| | **2** | 0 | No Growth | 0 | No Growth | 0 | No Growth |
| | **3** | 0 | No Growth | 0 | No Growth | 0 | No Growth |
| 2.5% cinnamon | **1** | 0 | No Growth | 0 | No Growth | 0 | No Growth |
| leaf oil | **2** | 0 | No Growth | 0 | No Growth | 0 | No Growth |
| | **3** | 0 | No Growth | 0 | No Growth | 0 | No Growth |
| 2.5% cinnamon bark oil | **1** | 20 | No Growth | 16 | No Growth | 0 | No Growth |
| | **2** | 21 | No Growth | 12 | No Growth | 0 | No Growth |
| | **3** | 20 | No Growth | 12 | No Growth | 0 | No Growth |
| 2.5% cinnamon bark oil | **1** | 24 | No Growth | 12 | No Growth | 0 | No Growth |
| | **2** | 20 | No Growth | 12 | No Growth | 0 | No Growth |
| | **3** | 19 | No Growth | 13 | No Growth | 0 | No Growth |
| 5% cinnamon bark oil | **1** | 19 | No Growth | 15 | No Growth | 32 | No Growth |
| | **2** | 21 | No Growth | 14 | No Growth | 31 | No Growth |
| | **3** | 19 | No Growth | 13 | No Growth | 36 | No Growth |
| 5% cinnamon bark oil | **1** | 13 | No Growth | 17 | No Growth | 34 | No Growth |
| | **2** | 14 | No Growth | 17 | No Growth | 38 | No Growth |
| | **3** | 13 | No Growth | 13 | No Growth | 35 | No Growth |
| 100% cinnamon bark oil | **1** | 40 | No Growth | 43 | No Growth | 32 | No Growth |
| | **2** | 40 | No Growth | 43 | No Growth | 31 | No Growth |
| | **3** | 38 | No Growth | 42 | No Growth | 30 | No Growth |
| 100% cinnamon bark oil | **1** | 50 | No Growth | 41 | No Growth | 33 | No Growth |
| | **2** | 46 | No Growth | 43 | No Growth | 36 | No Growth |
| | **3** | 45 | No Growth | 39 | No Growth | 35 | No Growth |
| CONTROL CEFAMASTER (cefalonium 8.3%) | **1** | 48 | No Growth | 52 | No Growth | 26 | No Growth |
| | **2** | 48 | No Growth | 54 | No Growth | 27 | No Growth |
| | **3** | 46 | No Growth | 56 | No Growth | 27 | No Growth |
| CONTROL CEFAMASTER (cefalonium 8.3%) | **1** | 47 | No Growth | 54 | No Growth | 26 | No Growth |
| | **2** | 48 | No Growth | 54 | No Growth | 28 | No Growth |
| | **3** | 48 | No Growth | 53 | No Growth | 26 | No Growth |
| CONTROL Carrier + Organism | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |

The Cinnamon Bark and Leaf Oil products showed activity against *Staphylococcus aureus* and *Streptococcus uberis* at both the 2.5 and 5% levels. The 5% Bark product also showed good activity against *E. Coli*. This activity was considered highly acceptable given that the rationale for inclusion of the Oil Extract is to help prevent ingress of new bacteria through or around the Teat Sealant material and therefore create a more effective barrier in the teat canal.

Test formulations were also tested under accelerated stress conditions. No physical separation of the constituents was observed when samples were exposed to temperatures of 55°C for 4 weeks.

### Example 4. Teat Sealant Formulations Containing Thyme Oil, Eucalyptus Oil and Aniseed Oil

Other potential oils known to have antibacterial properties were tested. These included thyme oil, eucalyptus oil and aniseed oil another plant based oil.

**Table 8: Antibacterial Activity of Teat Sealant Formulations**

| | | ***Staphylococcus aureus* ATCC6538** | | ***Streptococcus uberis* ATCC19436** | | ***Escherichia coli* ATCC8739** | |
|---|---|---|---|---|---|---|---|
| **Sample Description** | | **Zone Size (mm)** | **Growth/No Growth Under Aluminum Foil Carrier** | **Zone Size (mm)** | **Growth/No Growth Under Aluminum Foil Carrier** | **Zone Size (mm)** | **Growth/No Growth Under Aluminum Foil Carrier** |
| 5% thyme oil | **1** | 51 | No Growth | 30 | No Growth | 26 | No Growth |
| | **2** | 48 | No Growth | 29 | No Growth | 27 | No Growth |
| | **3** | 46 | No Growth | 33 | No Growth | 18 | No Growth |
| 5% thyme oil | **1** | 45 | No Growth | 30 | No Growth | 15 | No Growth |
| | **2** | 45 | No Growth | 35 | No Growth | 21 | No Growth |
| | **3** | 47 | No Growth | 31 | No Growth | 19 | No Growth |
| 5% eucalyptus oil | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |
| 5% eucalyptus oil | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |
| 5% aniseed oil | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |
| 5% aniseed oil | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |
| TEATSEAL® | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |
| TEATSEAL® | **1** | 0 | Growth | 0 | Growth | 0 | Growth |
| | **2** | 0 | Growth | 0 | Growth | 0 | Growth |
| | **3** | 0 | Growth | 0 | Growth | 0 | Growth |
| CEFAMASTER® (cefalonium 8.3%) | **1** | 44 | No Growth | 47 | No Growth | 27 | No Growth |
| | **2** | 43 | No Growth | 46 | No Growth | 27 | No Growth |
| | **3** | 41 | No Growth | 47 | No Growth | 28 | No Growth |
| CEFAMASTER® (cefalonium 8.3%) | **1** | 41 | No Growth | 46 | No Growth | 27 | No Growth |
| | **2** | 43 | No Growth | 46 | No Growth | 27 | No Growth |
| | **3** | 43 | No Growth | 48 | No Growth | 26 | No Growth |

Of the three oils tested thyme oil proved to be strongly inhibitory. Based on this surprising result, the reduced concentrations of thyme oil were tested to determine if a satisfactory level of activity could still be generated.

**Table 9: Antibacterial Activity of Thyme Oil Based Teat Sealant Formulations**

| | | ***Staphylococcus aureus* ATCC6538** | | ***Streptococcus uberis* ATCC19436** | | ***Escherichia coli* ATCC8739** | |
|---|---|---|---|---|---|---|---|
| **Sample Description** | | **Zone Size (mm)** | **Growth/No Growth Under Aluminum Foil Carrier** | **Zone Size (mm)** | **Growth/No Growth Under Aluminum Foil Carrier** | **Zone Size (mm)** | **Growth/No Growth Under Aluminum Foil Carrier** |
| 3% Thyme Oil | 1 | 28 | No Growth | 11 | No Growth | 11 | No Growth |
| | 2 | 31 | No Growth | 13 | No Growth | 11 | No Growth |
| | 3 | 31 | No Growth | 16 | No Growth | 11 | No Growth |
| 3% Thyme Oil | 1 | 31 | No Growth | 14 | No Growth | 11 | No Growth |
| | 2 | 34 | No Growth | 16 | No Growth | 11 | No Growth |
| | 3 | 33 | No Growth | 15 | No Growth | 11 | No Growth |
| 2% Thyme Oil | 1 | 19 | No Growth | 11 | No Growth | 0 | Growth |
| | 2 | 21 | No Growth | 11 | No Growth | 0 | Growth |
| | 3 | 18 | No Growth | 11 | No Growth | 0 | Growth |
| 2% Thyme Oil | 1 | 19 | No Growth | 0 | No Growth | 0 | Growth |
| | 2 | 18 | No Growth | 11 | No Growth | 0 | Growth |
| | 3 | 16 | No Growth | 0 | No Growth | 0 | Growth |
| 1% Thyme Oil | 1 | 11 | No Growth | 0 | Growth | 0 | Growth |
| | 2 | 13 | No Growth | 0 | Growth | 0 | Growth |
| | 3 | 11 | No Growth | 0 | Growth | 0 | Growth |
| 1% Thyme Oil | 1 | 14 | No Growth | 0 | Growth | 0 | Growth |
| | 2 | 13 | No Growth | 0 | Growth | 0 | Growth |
| | 3 | 16 | No Growth | 0 | Growth | 0 | Growth |
| CEFAMASTER® | 1 | 41 | No Growth | 30 | No Growth | 26 | No Growth |
| | 2 | 40 | No Growth | 31 | No Growth | 26 | No Growth |
| | 3 | 39 | No Growth | 29 | No Growth | 24 | No Growth |
| CEFAMASTER® | 1 | 43 | No Growth | 30 | No Growth | 22 | No Growth |
| | 2 | 41 | No Growth | 28 | No Growth | 26 | No Growth |
| | 3 | 40 | No Growth | 29 | No Growth | 24 | No Growth |

The result shows that in order to maintain a degree of inhibition against all three major mastitis pathogens it is desired to include a level of the thyme oil at a concentration higher than 2% in the chosen formulation base.

### Example 5. Retention Test of the Teat Sealant Formulations

Retention of the intramammary teat sealant in the udder of the cow is affected by a number of factors including viscosity. Batches of the formulation described herein were subject to viscosity testing at body temperature and compared with Pfizer TEATSEAL®. The result of this testing was that the base formulation described herein demonstrated a comparable viscosity to TEATSEAL®.

The formulation described herein incorporates about 65% w/w subnitrate and aluminum stearate gelling agent.

Samples of the formulation described herein and TEATSEAL® were also subject to an experiment in which 1g samples of each were placed in individual vertical tubes and heated to 40°C. The time taken for each sample to travel a 4cm distance down the tube was then recorded. The results were that the mean time taken for samples of TEATSEAL® was 49 minutes, while the mean time taken for samples of the formulation described herein was 53 minutes. The difference between the two products in this test was insignificant.

In field conditions retention of the formulation described herein can be expected to be comparable to TEATSEAL®. However, just as TEATSEAL® may fragment in some conditions or udders, there is a possibility this will occur with the formulation described herein. In these cases, the selected plant oil is expected to act as an additional barrier to the ingress of new bacterial infection since a significant portion of new intramammary bacterial infections in the dry period are due to *Streptococccus uberis ,Staphylococcus aureus or E. coli.*

While in the *in vitro* studies other oils proved to have some degree of inhibition it was decided to conduct more extensive testing with the 1% and 3% thyme oil formulations. The thyme oil used conformed to the European Pharmacopoeia:
*Description:* Essential oil obtained by steam distillation from the fresh flowering aerial parts of *Thymus vulgaris* L., *T. zygis* Loefl. ex L. or a mixture of both species.
*Appearance:* Clear, yellow or very dark reddish-brown, mobile liquid with a characteristic, aromatic, spicy odour, reminiscent of thymol.
*Solubilfty:* miscible with ethanol and with light petroleum.
*Key constituents:*
   *β-myrcene:* 1.0 per cent to 3.0 per cent,
      - *γ-terpinene*: 5.0 per cent to 10.0 per cent,
      - *p-cymene*: 15.0 per cent to 28.0 per cent,
      - *linalol*: 4.0 per cent to 6.5 per cent,
      - *terpinen-4-ol*: 0.2 per cent to 2.5 per cent,
      - *thymol*: 36.0 per cent to 55.0 per cent,
      - *carvacrol*: 1.0 per cent to 4.0 per cent.

Samples were prepared according to the following formulation;

**Table 10: Thyme Oil Based Teat Sealant Formulation**

| **Sr.No** | **Material** | **% w/w** |
|---|---|---|
| 1 | Liquid Paraffin Heavy | 26.7 |
| 2 | Alugel | 2.3 |
| 3 | Silicon dioxide | 1 |
| 4 | Bismuth Subnitrate (Heavy) | 65-67 |
| 6 | Thyme Oil | 1 - 3.0 |

A field study was undertaken to compare retention and pre/post gland swelling/pain between the commercially available TEATSEAL® non-antiinfective formulation and the two thyme oil based formulations (1% and 3% thyme oil).

### Retention of Thyme Oil based formulations in the teat canal

The duration over which TEATSEAL® and the two thyme oil based formulations persist in the teat canal following infusion was evaluated. Within each of 20 cows, the positive control formulation (i.e. TEATSEAL®) and one of the Thyme Oil formulations was infused in 2 glands each (Left Rear LR and Right Front RF for the thyme oil formulation and Left Front LF and Right Rear RR for the TEATSEAL® product). The cows were selected on the basis of having a maximum Somatic Cell Count in the preceding lactation of <200,000, no history of clinical mastitis and an expected dry period of > 60 days. Infusion of the teat sealant formulations occurred after the last milking of lactation and following aseptic preparation of the teat end. Prior to infusion the rectal temperature of the cow was determined and the mammary gland palpated for presence of any heat or swelling. At 15 minutes following infusion, the rectal temperature was again determined and the gland re-palpated.

The cows were presented for examination at 2, 4, 6, and 8 weeks after infusion and each gland of each cow assessed for the presence of swelling or pain upon palpation. At each time point, 5, 5 and 10 glands previously treated with 1% Thyme Oil, 3% Thyme Oil and TEATSEAL® respectively, were randomly selected and the teat sealant expressed. This selection was done such that only 1 gland per cow was expressed at each time point. The teat sealant was scored as present or absent and the milk from each quarter was put through filter paper and the teat sealant air-dried then weighed. Following expression of the teat sealant, the gland was again infused with the commercial TEATSEAL® formulation.

### Analysis

The proportion of glands which lost teat sealant was analysed using univariate χ² analysis with week and treatment as the independent variables. The weight of teat sealant was not normally distributed. Hence the log₁₀ of the weight (+1 to account for glands from which no teat sealant was found) was analysed. This data was analysed using a linear mixed model with week and treatment as the fixed effects and cow as a random effect with week within cow a repeated variable and using a compound symmetry covariance structure.

### Results

Following enrollment 10 cows were treated with 1% Thyme Oil (n = 20 glands) and TEATSEAL® and 11 cows with 3% Thyme Oil (n = 22 glands) and TEATSEAL®.

### Target Animal safety data pre and post infusion

The mean rectal temperature was 36.9°C (SEM = 0.2) and 37.6 °C (SEM = 0.1) before and at 15 minutes after infusion, respectively, with a maximum temperature of 38.6°C.

Slight swelling (score 2) was detected in 22 of the 84 glands examined pre infusion. One additional gland (in the TEATSEAL® group) was found to be swollen at 15 minutes after infusion (Table 11). Slight pain (score 2) was detected in 8 of 84 glands pre infusion and no glands post infusion (Table 11).

**Table 11. The number and percentage of glands which were scored as having swelling (score 2) or pain (score 2) before and at 15 minutes after infusion of 1 tube of 1% or 3% Thyme Oil based teat sealant formulations or commercial TEATSEAL® teat sealant.**

| | Treatment | Pre infusion | | | 15 minute post infusion | | |
|---|---|---|---|---|---|---|---|
| | | no. | total | % | no. | total | % |
| Swelling | 1% Thyme Oil | 9 | 20 | 45.0 | 9 | 20 | 45.0 |
| | 3% Thyme Oil | 2 | 22 | 9.1 | 2 | 22 | 9.1 |
| | TEATSEAL® | 11 | 42 | 26.2 | 12 | 42 | 28.6 |
| Pain | 1% Thyme Oil | 0 | 20 | 0.0 | 0 | 20 | 0.0 |
| | 3% Thyme Oil | 4 | 22 | 18.2 | 0 | 22 | 0.0 |
| | TEATSEAL® | 4 | 42 | 9.5 | 0 | 42 | 0.0 |

### Post infusion

A total of 12 glands were defined as having 'swelling' at some time point after treatment. All of these were score 2 (i.e. the lowest positive score). Five (6.2%), three (3.4%) and four (2.4%) of the total glands (glands x 4 time points) infused with 1% Thyme Oil, 3% Thyme Oil and TEATSEAL® were found to have swelling, respectively (Table 12).

Pain was detected in 2, 1, 1 and 1 of the LF, LR, RF and RR glands respectively, with a maximum of score 2 across all time points. One (1.2%), two (2.3%) and two (1.2%) of glands treated with 1% Thyme Oil, 3% Thyme Oil and TEATSEAL®, respectively, were detected with pain, all at week 4 (Table 12).

**Table 12. The number and percentage of glands defined as having swelling (i.e. score 2) or pain (i.e. score 2) when examined 2, 4, 6 and 8 weeks after infusion of 1% or 3% Thyme Oil or TEATSEAL®**

| | Week | Number of Glands | | | % of Glands at Time point | | |
|---|---|---|---|---|---|---|---|
| | | 1% Thyme Oil | 3% Thyme Oil | TEATSEAL | 1% Thyme Oil | 3% Thyme Oil | TEATSEAL |
| Swelling | 2 | 3 | 0 | 2 | 15.0 | 0.0 | 4.7 |
| | 4 | 2 | 2 | 2 | 10.0 | 9.0 | 4.7 |
| | 6 | 0 | 0 | 0 | 0.0 | 0.0 | 0.0 |
| | 8 | 0 | 1 | 0 | 0.0 | 4.5 | 0.0 |
| | Total | 5 | 3 | 4 | 6.2 | 3.4 | 2.4 |
| Pain | 2 | 0 | 0 | 0 | 0.0 | 0.0 | 0.0 |
| | 4 | 1 | 2 | 2 | 5.0 | 9.0 | 4.7 |
| | 6 | 0 | 0 | 0 | 0.0 | 0.0 | 0.0 |
| | 8 | 0 | 0 | 0 | 0.0 | 0.0 | 0.0 |
| | Total | 1 | 2 | 2 | 1.2 | 2.3 | 1.2 |

### Presence of teat sealant

There was no difference in proportion of glands from which teat sealant was lost over the time course of the study (p = 0.75). The loss rate was 4/20 (20%), 3/22 (14%) and 9/42 (21%) glands for the 1% Thyme Oil, 3% Thyme Oil and TEATSEAL® groups, respectively. The loss rate was not different between weeks with 19%, 14%, 24% and 19% of glands missing teat sealant material at weeks 2, 4, 6 and 8, respectively (p = 0.89).

### Weight of teat sealant

There was no difference in the mass of teat sealant between treatments (0.23 ± 0.06 vs. 0.20 ± 0.05 vs. 0.25 ± 0.04 (log₁₀ mean ± SEM) for 1% Thyme Oil, 3% Thyme Oil and TEATSEAL® groups respectively, p = 0.73). There was no effect of week (p = 0.79; Figure 1). There was also no treatment by week interaction (p = 0.14).

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention.

Having thus described in detail preferred embodiments of the present invention, it is to be understood that the invention defined by the above paragraphs is not to be limited to particular details set forth in the above description as many apparent variations thereof are possible without departing from the spirit or scope of the present invention.

Various preferred features and embodiments of the present invention will now be described with reference to the following numbered paragraphs (paras).
1. A teat sealant formulation comprising (a) one or more plant oil(s), (b) bismuth subnitrate, (c) liquid paraffin, (d) aluminum stearate, and (e) silicon dioxide, wherein the formulation is a gel or a paste.
2. The formulation of para 1, wherein said plant oil is selected from the group consisting of thyme oil, cinnamon bark oil and cinnamon leaf oil, or a combination thereof.
3. The formulation of paras 1 or 2, wherein said plant oil is present in an amount of about 1% to about 15% w/w.
4. The formulation of any one of paras 1, 2 or 3, wherein said bismuth subnitrate is present in an amount of about 40% to about 90%.
5. The formulation of any one of paras 1, 2, 3 or 4, wherein said liquid paraffin is present in an amount of about 5% to about 50% w/w.
6. The formulation of paras 1-5, wherein said aluminum stearate is present in an amount of about 0.1, % to about 10% w/w.
7. The formulation of any one of paras 1-6, wherein said silicon dioxide is present in an amount of about 0.1%, about 0.2%, about 0.3%, about 0.4%, about 0.5%, about 0.6%, about 0.7%, about 0.8%, about 0.9%, about 1%, about 2%, about 3%, about 4% or about 5% w/w.
8. The formulation of para, 1, wherein said (a) plant oil is present in an amount of about 1% to about 10% w/w, (b) bismuth subnitrate is present in an amount of about 55% to about 75%, (c) liquid paraffin is present in an amount of about 20% to about 35% w/w, (d) aluminum stearate is present in an amount of about 2% to about 3% w/w, and (e) silicon dioxide is present in an amount of about 1% w/w.
9. The formulation of any one of paras 1, 3, 4, 5, 6, 7 or 8, wherein said plant oil is selected from the group consisting of thyme oil, cinnamon bark oil and cinnamon leaf oil, or a combination thereof.
10. A teat sealant formulation comprising one or more plant oil and bismuth subnitrate, wherein said formulation is a gel or a paste.
11. The teat sealant formulation of para 10, wherein said formulation further comprises liquid paraffin, aluminum stearate, or silicon dioxide.
12. The use of the formulation of any one of paras 1-11 as a medicament for treating an animal to prevent or treat mastitis, comprising administering to the animal said formulation, wherein said formulation is a gel or a paste.
13. The use of para 12, wherein said animal is a livestock animal.
14. A method for manufacturing a medicament intended for treating mastitis in an animal, characterized in that a formulations of any one of paras 1-11 is used.
15. The method of para 14, wherein said animal is a livestock animal.

## Claims

1. A teat sealant formulation comprising (a) one or more plant oil(s), (b) bismuth subnitrate, (c) liquid paraffin, (d) aluminum stearate, and (e) silicon dioxide, wherein the formulation is a gel or a paste; wherein said plant oil is selected from the group consisting of cinnamon bark oil, cinnamon leaf oil, and thyme oil, or a combination thereof.

2. The formulation of claim 1, wherein said plant oil is present in an amount of about 1% to about 15% w/w.

3. The formulation of claim 1 or 2, wherein said bismuth subnitrate is present in an amount of about 40% to about 90% w/w.

4. The formulation of any one of claims 1, 2 or 3, wherein said liquid paraffin is present in an amount of about 5% to about 50% w/w.

5. The formulation of claims 1 to 4, wherein said aluminum stearate is present in an amount of about 0.1 % to about 10% w/w.

6. The formulation of any one of claims 1 to 5, wherein said silicon dioxide is present in an amount of about 0.1%, about 0.2% , about 0.3%, about 0.4%, about 0.5%, about 0.6%, about 0.7%, about 0.8%, about 0.9%, about 1%, about 2%, about 3%, about 4% or about 5% w/w.

7. The formulation of claim 1, wherein said (a) plant oil is present in an amount of about 1% to about 10% w/w, (b) bismuth subnitrate is present in an amount of about 55% to about 75%, (c) liquid paraffin is present in an amount of about 20% to about 35% w/w, (d) aluminum stearate is present in an amount of about 2% to about 3% w/w, and (e) silicon dioxide is present in an amount of about 1% w/w.

8. The formulation of any one of claims 1 to 7 for use in controlling the growth of *Staphylococcus aureus, Streptococcus uberis* and/or *E. coli.*

9. The use of the formulation of any one of claims 1 to 7 in the manufacture of a medicament for the prevention or treatment of mastitis in an animal.

10. The use of claim 9, wherein said animal is a livestock animal.

11. The use according to claim 8 or 9 wherein the formulation controls the growth of *Staphylococcus aureus, Streptococcus uberis* and/or *E. coli.*

12. A method for manufacturing a medicament intended for treating mastitis in an animal, wherein said method comprises contacting or mixing the components of the formulation as detailed in any one of claims 1 to 7.

13. The method of claim 12, wherein said animal is a livestock animal.

14. The formulation of any one of claims 1 to 7 for use in the prevention of treatment of mastitis in an animal, preferably wherein said animal is a livestock animal.

15. The formulation according to claim 14 wherein the use comprises controlling the growth of *Staphylococcus aureus, Streptococcus uberis* and/or *E. coli.*
